# EUROPEAN PATENT APPLICATION

(11) **EP 4 768 919 A1**
(43) Date of publication of application: **01.07.2026**
(21) Application number: 25227575.5
(22) Date of filing: 30.12.2025
(51) Int. Cl.: G01N 33/68

(54) **USE OF N-TERMINAL FRAGMENT OF IGFBP-4 IN CLINICAL ASSESSMENT OF HEART FAILURE AND CORONARY HEART DISEASE**

(30) Priority: 30.12.2024 CN 202411990243
(71) Applicant: Shenzhen Mindray Bio-Medical Electronics Co., Ltd., Shenzhen, Guangdong 518057 (CN)
(72) Inventor: ZHANG, Yi, Shenzhen 518057 (CN); WANG, Jing, Shenzhen 518057 (CN); LIU, Junjun, Shenzhen 518057 (CN); LI, Ke, Shenzhen 518057 (CN)
(74) Representative: KIPA AB

(57) **Abstract**

The present disclosure relates to the technical field of biomedicine, in particular the use of an N-terminal fragment of insulin-like growth factor binding protein-4 in the clinical assessment of heart failure and coronary heart disease.

## Description

### CROSS REFERENCE

This application claims benefit of priority of Chinese Patent Application No. 202411990243.4 filed on December 30, 2024, the content of which is incorporated herein by reference in its entirety.

### TECHNICAL FIELD

The present disclosure relates to the technical field of biomedicine, in particular to the use of N-terminal fragment of IGFBP-4 in the clinical assessment of heart failure and coronary heart disease.

### BACKGROUND

Heart failure (abbreviated as "HF") is a complex clinical syndrome resulting from abnormal changes in cardiac structure or function, leading to an absolute reduction or relative insufficiency of cardiac output that fails to meet the needs of the body's tissue cell, representing the most severe end-stage of cardiovascular diseases. With the change of living habits in modern society and the aggravation of population aging, the prevalence of heart failure continues to rise, and its high disability and mortality rates have become a major global public health problem.

The clinical manifestations of heart failure are diverse, including dyspnoea, fatigue, oedema, etc. Its diagnosis primarily relies on medical history, symptoms, signs, and auxiliary examinations, where the auxiliary examinations involve chest X-ray, detection of marker, and ultrasound cardiogram to confirm the presence of heart failure. Among these, ultrasound cardiogram is the preferred method for assessing cardiac structure and function. The left ventricular ejection fraction (LVEF) measured by ultrasound cardiogram, shows the amount of blood pumped out by the left ventricle during each contraction of the heart. Based on the ejection fraction (EF) level, heart failure can be classified as heart failure with reduced ejection fraction HFrEF (LVEF < 40%), heart failure with preserved ejection fraction HFpEF (LVEF ≥ 50%), and heart failure with mid-range ejection fraction HFmrEF (LVEF between 40% to 49%). However, by the time heart failure can be definitely diagnosed clinically, the abnormal cardiac structure or function has often undergone irreversible and profound progression, and therefore there is an urgent need for a clinical application method that is capable of assisting heart failure risk at an early stage.

### SUMMARY

An object of the present disclosure is to provide the use of amino-terminal (hereinafter abbreviated as N-terminal) fragment of insulin-like growth factor binding protein-4 (hereinafter abbreviated as IGFBP-4) in the clinical assessment of heart failure and coronary heart disease.

For this purpose, in a first aspect, the present disclosure provides a method for diagnosing or assisting in the diagnosis of heart failure in a subject, wherein the method comprises:
detecting a level of N-terminal fragment of insulin-like growth factor binding protein-4 in a sample from the subject, wherein the N-terminal fragment of insulin-like growth factor binding protein-4 is an N-terminal fragment obtained by catalytic hydrolysis of insulin-like growth factor binding protein-4 by pregnancy-associated plasma protein A;
diagnosing or assisting in the diagnosis of heart failure based on the level of N-terminal fragment of insulin-like growth factor binding protein-4.

In some embodiments, the diagnosing or assisting in the diagnosis of heart failure comprises:
(i) diagnosing or assisting in the diagnosis of whether a subject has heart failure; or
(ii) diagnosing or assisting in the diagnosis of a severity of heart failure in a subject; preferably, the severity refers to left ventricular ejection fraction or NYHA cardiac function classification.

In some embodiments, the method is for diagnosing or assisting in the diagnosis of whether a subject has heart failure, and wherein:
comparing the level of the N-terminal fragment of insulin-like growth factor binding protein-4 with a reference value, wherein
a level of the N-terminal fragment of insulin-like growth factor binding protein-4 higher than the reference value indicates that the subject has heart failure;
a level of the N-terminal fragment of insulin-like growth factor binding protein-4 lower than the reference value indicates that the subject does not have heart failure.

In some embodiments, the reference value is about 148.5 ng/mL.

In some embodiments, the method is for diagnosing or assisting in the diagnosis of a severity of heart failure, and wherein higher level of NT-IGFBP-4 indicates a lower left ventricular ejection fraction (LVEF%) and/or a higher NYHA cardiac function classification score in the subject.

In some embodiments, the method further comprises:
detecting a level of N-terminal pro-brain natriuretic peptide in a sample from the subject;
diagnosing or assisting in the diagnosis of heart failure based on the level of the N-terminal fragment of insulin-like growth factor binding protein-4 and the level of the N-terminal pro-brain natriuretic peptide.

In some embodiments, the method is for diagnosing or assisting in the diagnosis of whether a subject has heart failure, and comprising:
comparing the level of the N-terminal fragment of IGFBP-4 and the level of the NT-proBNP with their respective reference values, and wherein:
when the level of the NT-proBNP is lower than its reference value, it indicates that the subject does not have heart failure;
when the level of the NT-proBNP is higher than its reference value, and the level of the N-terminal fragment of IGFBP-4 is lower than its reference value, it indicates that the subject is at risk of heart failure and requires further determination by other clinical diagnostic means (e.g., chest X-ray, electrocardiogram, and/or ultrasound cardiogram);
when the level of the N-terminal fragment of IGFBP-4 and the level of the NT-proBNP are both higher than their respective reference values, it indicates that the subject has heart failure.

In some embodiments, the reference value of NT-proBNP is about 125 ng/L.

In some embodiments, the reference value of NT-IGFBP-4 is about 220 ng/mL.

In some embodiments, the method further comprises administering a drug to the subject diagnosed with heart failure, and wherein the drug comprises Angiotensin Converting Enzyme Inhibitors (ACEI) (e.g., Captopril, Enalapril), Angiotensin Receptor Blockers (ARB) (e.g., Valsartan, Losartan), Angiotensin Receptor Neprilysin Inhibitor (ARNI) (e.g., Sacubitril/Valsartan), Beta-blockers (e.g., Metoprolol, Bisoprolol, Carvedilol), Aldosterone Receptor Antagonists (e.g., Spironolactone, Eplerenone), Diuretics (e.g., Furosemide, Hydrochlorothiazide, Spironolactone), SGLT2 Inhibitors (e.g., Dapagliflozin, Empagliflozin), Positive Inotropic Agents (e.g., Digoxin), or Sinus Node Inhibitors (e.g., Ivabradine).

In some embodiments, the method further comprises administering the above drug to the subject with the level of the N-terminal fragment of IGFBP-4 and the level of the NT-proBNP both higher than their respective reference values, without additional determination by other clinical diagnostic means (e.g., chest X-ray, electrocardiogram, and/or ultrasound cardiogram).

In some embodiments, the level of N-terminal fragment of insulin-like growth factor binding protein-4 is detected by double-antibody sandwich immunoassay (e.g., double-antibody sandwich chemiluminescence immunoassay) comprising a capture antibody and a detection antibody, and wherein one of the capture antibody and the detection antibody is an antibody that specifically binds to NT-IGFBP-4, and the other is capable of recognizing and binding to NT-IGFBP-4 or full-length IGFBP-4.

In some embodiments, the detection antibody bears a detection label, e.g., radioactive label, fluorescent label, chemiluminescent label, biotin, colloidal gold, electrochemiluminescent label, quantum dot or enzyme. In some embodiments, the detection label is selected from enzyme, e.g., alkaline phosphatase.

In some embodiments, the capture antibody is coated on the surface of a solid carrier, e.g., magnetic bead or microtiter plate. In some embodiments, the capture antibody is coated on the surface of a magnetic bead.

In some embodiments, the subject shows clinical manifestations such as dyspnoea, chest tightness, wheezing, shortness of breath, and other suspected heart failure symptoms. In some embodiments, the subject shows no clinical manifestations.

In a second aspect, the present disclosure provides a method for assessing or assisting in the assessment of prognostic risk of heart failure in a subject, wherein the method comprises:
detecting a level of N-terminal fragment of insulin-like growth factor binding protein-4 in a sample from the subject, wherein the N-terminal fragment of insulin-like growth factor binding protein-4 is an N-terminal fragment obtained by catalytic hydrolysis of insulin-like growth factor binding protein-4 by pregnancy-associated plasma protein A;
assessing or assisting in the assessment of prognostic risk of heart failure based on the level of N-terminal fragment of insulin-like growth factor binding protein-4.

In some embodiments, the assessing or assisting in the assessment of prognostic risk of heart failure comprises:
(iii) assessing or assisting in the assessment of short-term prognostic risk in a heart failure patient, the short-term referring to a period less than 30 days; or
(iv) assessing or assisting in the assessment of long-term prognostic risk in a heart failure patient, the long-term referring to a period more than 30 days and less than 240 days; preferably, the prognostic risk refers to the risk of cardiac-related rehospitalization and/or death.

In some embodiments, the method comprising comparing the level of the N-terminal fragment of insulin-like growth factor binding protein-4 with a reference value, wherein
a level of the N-terminal fragment of insulin-like growth factor binding protein-4 higher than the reference value indicates that the subject has a high risk of poor prognosis for heart failure;
a level of the N-terminal fragment of insulin-like growth factor binding protein-4 lower than the reference value indicates that the subject has a low risk of poor prognosis for heart failure.

In some embodiments, the reference value for short-term prognostic risk is about 314 ng/mL.

In some embodiments, the reference value for long-term prognostic risk is about 283 ng/mL.

In some embodiments, the method further comprises:
detecting a level of N-terminal pro-brain natriuretic peptide in a sample from the subject;
assessing or assisting in the assessment of prognostic risk of heart failure based on the level of the N-terminal fragment of insulin-like growth factor binding protein-4 and the level of the N-terminal pro-brain natriuretic peptide.

In some embodiments, the method comprising:
comparing the level of the N-terminal fragment of IGFBP-4 and the level of the NT-proBNP with their respective reference values, wherein:
when the level of the N-terminal fragment of IGFBP-4 and the level of the NT-proBNP are both higher than their respective reference values, it indicates that the subject has a high risk of poor prognosis for heart failure;
when only one of the level of the N-terminal fragment of IGFBP-4 and the level of the NT-proBNP is higher than its respective reference value, it indicates that the subject has a moderate risk of poor prognosis for heart failure;
when the level of the N-terminal fragment of IGFBP-4 and the level of the NT-proBNP are both lower than their respective reference values, it indicates that the subject has a low risk of poor prognosis for heart failure.

In some embodiments, the reference value of NT-proBNP for short-term prognostic risk is about 3110 ng/L. In some embodiments, the reference value of NT-IGFBP-4 for short-term prognostic risk is about 314 ng/mL.

In some embodiments, the reference value of NT-proBNP for long-term prognostic risk is about 3110 ng/L. In some embodiments, the reference value of NT-IGFBP-4 for long-term prognostic risk is about 283 ng/mL.

In some embodiments, the method further comprising hospitalizing the subject identified as having a high risk of poor prognosis for heart failure (e.g., the subject having the level of the N-terminal fragment of IGFBP-4 and the level of the NT-proBNP both higher than their respective reference values).

In some embodiments, the level of N-terminal fragment of insulin-like growth factor binding protein-4 is detected by double-antibody sandwich immunoassay (e.g., double-antibody sandwich chemiluminescence immunoassay) comprising a capture antibody and a detection antibody, and wherein one of the capture antibody and the detection antibody is an antibody that specifically binds to NT-IGFBP-4, and the other is capable of recognizing and binding to NT-IGFBP-4 or full-length IGFBP-4.

In some embodiments, the detection antibody bears a detection label, e.g., radioactive label, fluorescent label, chemiluminescent label, biotin, colloidal gold, electrochemiluminescent label, quantum dot or enzyme. In some embodiments, the detection label is selected from enzyme, e.g., alkaline phosphatase.

In some embodiments, the capture antibody is coated on the surface of a solid carrier, e.g., magnetic bead or microtiter plate. In some embodiments, the capture antibody is coated on the surface of a magnetic bead.

In some embodiments, the subject has acute exacerbation of chronic heart failure.

In some embodiments, the subject is diagnosed with heart failure.

In a third aspect, the present disclosure provides a method for assessing or assisting in the assessment of prognostic risk of coronary heart disease, wherein the method comprises:
detecting a level of N-terminal fragment of insulin-like growth factor binding protein-4 in a sample from a coronary heart disease patient, wherein the N-terminal fragment of insulin-like growth factor binding protein-4 is the N-terminal fragment obtained by catalytic hydrolysis of insulin-like growth factor binding protein-4 by pregnancy-associated plasma protein A;
assessing or assisting in the assessment of prognostic risk of coronary heart disease based on the level of N-terminal fragment of insulin-like growth factor binding protein-4.

In some embodiments, the method comprising comparing the level of the N-terminal fragment of insulin-like growth factor binding protein-4 with a reference value, wherein
a level of the N-terminal fragment of insulin-like growth factor binding protein-4 higher than the reference value indicates that the subject has a high risk of poor prognosis for coronary heart disease;
a level of the N-terminal fragment of insulin-like growth factor binding protein-4 lower than the reference value indicates that the subject has a low risk of poor prognosis for coronary heart disease.

In some embodiments, the reference value is about 174 ng/mL.

In some embodiments, the level of N-terminal fragment of insulin-like growth factor binding protein-4 is detected by double-antibody sandwich immunoassay (e.g., double-antibody sandwich chemiluminescence immunoassay) comprising a capture antibody and a detection antibody, and wherein one of the capture antibody and the detection antibody is an antibody that specifically binds to NT-IGFBP-4, and the other is capable of recognizing and binding to NT-IGFBP-4 or full-length IGFBP-4.

In some embodiments, the detection antibody bears a detection label, e.g., radioactive label, fluorescent label, chemiluminescent label, biotin, colloidal gold, electrochemiluminescent label, quantum dot or enzyme. In some embodiments, the detection label is selected from enzyme, e.g., alkaline phosphatase.

In some embodiments, the capture antibody is coated on the surface of a solid carrier, e.g., magnetic bead or microtiter plate. In some embodiments, the capture antibody is coated on the surface of a magnetic bead.

In some embodiments, the method further comprising hospitalizing the subject identified as having a high risk of poor prognosis for coronary heart disease (e.g., the subject having the level of the N-terminal fragment of IGFBP-4 higher than its reference value).

In some embodiments, the subject is diagnosed with coronary heart disease.

In a fourth aspect, the present disclosure provides a kit comprising a first reagent for detecting N-terminal fragment of insulin-like growth factor binding protein-4 and a second reagent for detecting N-terminal pro-brain natriuretic peptide.

In some embodiments, the first reagent is for double-antibody sandwich immunoassay (e.g., double-antibody sandwich chemiluminescence immunoassay) and comprises a capture antibody and a detection antibody, and wherein one of the capture antibody and the detection antibody is an antibody that specifically binds to NT-IGFBP-4, and the other is capable of recognizing and binding to NT-IGFBP-4 or full-length IGFBP-4.

In some embodiments, the second reagent is for immunoassay (e.g., enzyme-linked immunoassay, radioimmunoassay, fluorescent immunoassay, chemiluminescence immunoassay). In some embodiments, the second reagent is for double-antibody sandwich immunoassay (e.g., double-antibody sandwich ELISA or CLIA) and comprises a capture antibody and a detection antibody.

In some embodiments, the detection antibody bears a detection label, e.g., radioactive label, fluorescent label, chemiluminescent label, biotin, colloidal gold, electrochemiluminescent label, quantum dot or enzyme. In some embodiments, the detection label is selected from enzyme, e.g., alkaline phosphatase.

In some embodiments, the capture antibody is coated on the surface of a solid carrier, e.g., magnetic bead or microtiter plate. In some embodiments, the capture antibody is coated on the surface of a magnetic bead.

In some embodiments of any of the above aspects, the N-terminal fragment of IGFBP-4 includes an amino acid sequence as set forth in SEQ ID NO: 1.

In some embodiments of any of the above aspects, detecting the level of the N-terminal fragment of insulin-like growth factor binding protein-4 in a sample from the subject comprises the following steps:
providing a reagent, wherein the reagent comprises a magnetic solid phase carrier coated with a capture antibody, and an alkaline phosphatase-labelled detection antibody, wherein at least one of the capture antibody and the detection antibody is capable of specifically binding to the N-terminal fragment of insulin-like growth factor binding protein-4;
providing a sample from the subject;
mixing the sample with the magnetic solid phase carrier coated with the capture antibody and the alkaline phosphatase-labelled detection antibody, and incubating the mixture to form an immune complex;
providing a magnetic field condition, and washing the immune complex to remove unbound substances;
adding a chemiluminescent substrate to the washed immune complex, wherein the chemiluminescent substrate is suitable for being decomposed by alkaline phosphatase to produce chemiluminescence; detecting intensity of the chemiluminescence, and calculating the level of the N-terminal fragment of insulin-like growth factor binding protein-4.

The above description is merely a summary of the technical solutions of the present disclosure. To make the technical means of the present disclosure more clearly understood and can be implemented according to the content of the description, and to make the above and other objects, features, and advantages of the present disclosure more apparent and comprehensible, specific embodiments of the present disclosure are listed below.

### BRIEF DESCRIPTION OF THE DRAWINGS

By reading the detailed description of the preferred embodiments below, various additional advantages and benefits will become apparent to those of ordinary skill in the art. The accompanying drawings are only for the purpose of illustrating preferred embodiments and are not considered to be a limitation of the present disclosure. In the accompanying drawings:
FIG. 1: Sample inclusion flowchart for healthy population and heart failure population;
FIG. 2: Concentration levels of NT-IGFBP-4 in healthy population and heart failure population;
FIG. 3: Analysis flowchart for heart failure diagnostic performance of NT-IGFBP-4 kit;
FIG. 4: ROC curve of heart failure diagnosis using NT-IGFBP-4;
FIG. 5: Diagnostic decision tree for combined use of NT-IGFBP-4 and NT-proBNP;
FIG. 6: Correlation analysis between NT-IGFBP-4 concentration level and left ventricular ejection fraction (LVEF%);
FIG. 7: Correlation analysis between NT-IGFBP-4 concentration level and NYHA cardiac function classification score;
FIG. 8: Inclusion and exclusion flowchart for heart failure prognosis study samples;
FIG. 9: Kaplan-Meier survival curves of heart failure populations within 240-day follow-up duration when the prognostic risk of heart failure is assessed using NT-IGFBP-4 alone, where the upper curve represents the low-risk group, and the lower curve represents the high-risk group;
FIG. 10: Kaplan-Meier survival curves of heart failure populations within 240-day follow-up duration when the prognostic risk of heart failure is assessed using a combination of NT-IGFBP-4 and NT-proBNP, where the three curves, from top to bottom, represent: the low-risk group, the moderate-risk group, and the high-risk group;
FIG. 11: Kaplan-Meier survival curves of heart failure populations within 30-day follow-up duration when the prognostic risk of heart failure is assessed using NT-IGFBP-4 alone, where the upper curve represents the low-risk group, and the lower curve represents the high-risk group;
FIG. 12: Kaplan-Meier survival curves of heart failure populations within 30-day follow-up duration when the prognostic risk of heart failure is assessed using NT-proBNP alone, where the upper curve represents the low-risk group, and the lower curve represents the high-risk group;
FIG. 13: Kaplan-Meier survival curves of heart failure populations within 30-day follow-up duration when the prognostic risk of heart failure is assessed using a combination of NT-IGFBP-4 and NT-proBNP, where the three curves, from top to bottom, represent: the low-risk group, the moderate-risk group, and the high-risk group;
FIG. 14: Inclusion and exclusion flowchart for coronary heart disease prognosis study samples;
FIG. 15: Kaplan-Meier survival curves of coronary heart disease populations within 120-day follow-up duration when the prognostic risk of coronary heart disease is assessed using NT-IGFBP-4, where the upper curve represents the low-risk group, and the lower curve represents the high-risk group.

### DETAILED DESCRIPTION

Exemplary embodiments of the present disclosure will be described in more detail below with reference to the accompanying drawings. While exemplary embodiments of the present disclosure are shown in the accompanying drawings, it should be understood that the present disclosure may be implemented in various forms and should not be limited by the embodiments set forth herein. Rather, these embodiments are provided so that this disclosure can be understood more thoroughly and the scope of this disclosure can be fully communicated to those skilled in the field.

### Definitions

As used herein, the terms "amino-terminal fragment of insulin-like growth factor binding protein-4", "N-terminal fragment of IGFBP-4", and "NT-IGFBP-4" have the same meaning. IGFBP-4 is a member of the insulin-like growth factor binding protein (IGFBP) superfamily. The human IGFBP-4 gene is located on human chromosome 17P13-12, has a full length of 15.3 kb, and consists of 4 exons and 3 introns. Human IGFBP-4 consists of 237 amino acids (e.g., aa 22-258 of GenBank: KAI4049325.1), and the primary product contains a signal peptide consisting of 21 amino acids. Under physiological conditions *in vivo,* IGFBP-4 can be cleaved by pregnancy-associated plasma protein-A (PAPP-A) at a specific site, releasing the N-terminal fragment of IGFBP-4 and the C-terminal fragment of IGFBP-4 into the blood circulation. The N-terminal fragment of human IGFBP-4 has the amino acid sequence as set forth in SEQ ID NO: 1.

SEQ ID NO: 1
DEAIHCPPCSEEKLARCRPPVGCEELVREPGCGCCATCALGLGMPCGVYT
PRCGSGLRCYPPRGVEKPLHTLMHGQGVCMELAEIEAIQESLQPSDKDEGDH
PNNSFSPCSAHDRRCLQKHFAKIRDRSTSGGKM

As used herein, the terms "heart failure" and "HF" have the same meaning, which is a clinical syndrome caused by various cardiac structural or functional diseases that impair ventricular filling and ejection function, resulting in cardiac output insufficient to meet the metabolic demands of body tissues. This condition is mainly characterized by symptoms such as dyspnoea, limited physical activity, and fluid retention, and features high mortality and poor prognosis.

As used herein, the terms "amino-terminal pro-brain natriuretic peptide", "N-terminal pro-brain natriuretic peptide" and "NT-proBNP" have the same meaning, which is a single peptide having a linear structure secreted by the ventricles. When cardiomyocytes are stimulated, a precursor pro-B-type natriuretic peptide (pre-proBNP) containing 134 amino acids is produced, followed by formation of pro-B-type natriuretic peptide (proBNP) containing 108 amino acids; the latter is further cleaved by an endopeptidase into NT-proBNP and BNP. Due to its longer half-life and relative stability in vivo, NT-proBNP has become a diagnostic biomarker endorsed by clinical guidelines for heart failure. The method for detecting NT-proBNP is well-known to a person skilled in the art, and commercially available kits have been provided, see e.g., NT-proBNP assay (Mindray, #105-027045-00), Human NT proBNP ELISA Kit (Abcam, #ab263877), Human proBNP/NPPB ELISA Kit (Invitrogen, #EHPRONPPB), LumiraDx NT-proBNP Test (Roche, #10418320001).

As used herein, the term "specifically binding to N-terminal fragment of IGFBP-4" means that an antibody or an antigen-binding fragment thereof is capable of recognizing and binding to N-terminal fragment of IGFBP-4, and has substantially no cross-reactivity with other proteins (such as full-length IGFBP-4 and C-terminal fragment of IGFBP-4). In some embodiments, an epitope recognized by the antibody that specifically binds to NT-IGFBP-4 includes a neoepitope formed at the N-terminal end of IGFBP-4 after cleavage of full-length IGFBP-4 by pregnancy-associated plasma protein-A (PAPP-A), where the full-length IGFBP-4 does not include the neoepitope. For example, in some embodiments, an antibody or an antigen-binding fragment thereof that specifically binds to N-terminal fragment of IGFBP-4 has less than 5% cross-reactivity with full-length IGFBP-4.

As used herein, the term "coronary heart disease" refers to coronary atherosclerotic heart disease, which is a heart disease caused by lesions such as atherosclerosis, thromboembolism, or vasospasm in the coronary arteries, leading to luminal narrowing or even occlusion, which in turn results in myocardial ischemia, hypoxia, or necrosis.

As used herein, the terms "patient" and "subject" refer to an organism, such as a mammal. In some embodiments, the subject is a human.

As used herein, the term "sample" refers to a substance obtained from a subject or isolated tissue, cell, or body fluid suitable for detection of a marker (such as NT-IGFBP-4). In some embodiments, the sample may be blood, serum, plasma, cerebrospinal fluid, lymph fluid, spinal fluid, mucosal secretion, exudate, amniotic fluid, synovial fluid, peritoneal fluid, etc.

As used herein, the term "ROC curve" refers to a receiver operating characteristic curve. In certain specific embodiments of the present disclosure, the ROC curve refers to an ROC curve between the true positive rate and the false positive rate. The area under the curve of ROC is the AUC. The ROC curve and the AUC are commonly used to evaluate the performance of clinical assessment.

As used herein, the terms "high expression" and "higher than a reference value" have the same meaning, referring to an increase of at least 5%, 10%, 20%, 30%, 50%, 80%, 100% or more compared with a "reference value" or "threshold".

As used in the present disclosure, the terms "low expression" and "lower than a reference value" have the same meaning, referring to a decrease of at least 5%, 10%, 20%, 30%, 50%, 80% or more compared with a "reference value" or "threshold".

As used in the present disclosure, a "reference value" or "threshold" for gene or protein expression may be set by those skilled in the art based on the gist of the present disclosure. In some embodiments, selecting an appropriate "reference value" or "threshold" comprises the following steps: a statistically significant analysis may first be performed based on the corresponding protein expression level in samples from subjects (patients) with a confirmed diagnosis or a definite prognosis , and the obtained expression value is used as a "reference value" or "threshold". Alternatively, in some embodiments, a threshold recommended by clinical guidelines may be used for certain markers such as NT-proBNP.

As used herein, the term "antibody" refers to an immunoglobulin molecule that can specifically bind to a target via at least one antigen recognition site located in the variable region of the immunoglobulin molecule. The "antibody" used herein includes not only complete (i.e., full-length) antibodies, but also antigen-binding fragments thereof (e.g., Fab, Fab', F(ab')₂, Fv, scFv, Fd, CDR fragments), variants thereof, fusion proteins comprising antibodies, humanized antibodies, chimeric antibodies, double antibodies, linear antibodies, single-chain antibodies, multispecific antibodies (e.g., bispecific antibodies), and any other modified configurations of immunoglobulin molecules containing antigen recognition sites of desired specificity, including antibody glycosylation variants, antibody amino acid sequence variants, and covalently modified antibodies. Antibodies can be derived from any mammals, including but not limited to humans, monkeys, pigs, horses, rabbits, dogs, cats, rats, mice, etc., or other animals such as birds (e.g., chickens), fish (e.g., sharks), and camels (e.g., llamas).

Typically, a complete or full-length antibody contains two heavy chains and two light chains. Each heavy chain contains a heavy chain variable region (VH) and a heavy chain constant region (CH); each light chain contains a light chain variable region (VL) and a light chain constant region (CL). Full-length antibodies can be any class of antibodies, such as IgD, IgE, IgG, IgA, or IgM (or subclasses of the above), but antibodies do not need to belong to any particular class. Immunoglobulins can be assigned to different classes based on their antibody heavy chain constant region amino acid sequences. Typically, there are five major classes of immunoglobulins: IgA, IgD, IgE, IgG, and IgM, and several of these classes can be further divided into subclasses, such as IgG1, IgG2, IgG3, IgG4, IgA1, and IgA2.

As used herein, the term "antigen-binding fragment" or "antigen-binding portion" refers to a portion or region of a complete antibody molecule that is responsible for binding to an antigen. The antigen-binding portion may comprise a heavy chain variable region (VH), a light chain variable region (VL), or both. Each of VH and VL typically comprises three complementary determining regions CDR1, CDR2, and CDR3. Nonlimiting examples of antigen-binding fragment include Fab, Fab', F(ab')₂, Fd, Fv, complementary determining region (CDR) fragments, scFv, diabody, single domain antibody, chimeric antibody, linear antibody, nanobody (technology from Domantis), probody, and such polypeptides, which comprises at least a portion of antibody that is sufficient to confer the specific antigen binding ability to the polypeptide.

As used herein, the term "immunological assay" refers to an assay that utilizes the specific interaction/binding affinity between antigen and antibody, which can generally be used to detect the presence or level of a specific antigen or antibody in a sample. Such immunological assays are well known to those skilled in the art and include, but are not limited to, enzyme immunoassay (EIA), chemiluminescence immunoassay (CLIA), radioimmunoassay (RIA), fluorescence immunoassay (FIA), Western blotting, immunoturbidimetric method, surface plasmon resonance method, etc.

As used herein, the term "double antibody sandwich method" specifically refers to an immunoassay technique commonly used in which an antigen is bound to two different antibodies. The antibodies separately bind to two different epitopes in the antigen that do not overlap or interfere. In this assay, a sandwich comprising a capture antibody, an antigen, and a detection antibody is formed, whereby the antigen bridges the two antibodies bound thereto. In some embodiments, the detection antibody bears a detection label, e.g., radioactive label, fluorescent label, chemiluminescent label, biotin, colloidal gold, electrochemiluminescent label, quantum dot or enzyme. In some embodiments, the detection label is selected from enzyme, e.g., alkaline phosphatase. In some embodiments, the capture antibody is coated on the surface of a solid carrier, e.g., magnetic bead or microtiter plate. In some embodiments, the capture antibody is coated on the surface of a magnetic bead.

As used herein, the term "about" means ±10% (e.g., ±5%, ±2%, or ±1%) of the indicated range or value, unless otherwise indicated. It should be understood that the terms "a" and "an" as used herein refer to "one or more" of the enumerated components. The use of the alternative (e.g., "or") should be understood to mean either one, both, or any combination thereof of the alternatives.

### Product and method for diagnosing or assisting in the diagnosis of heart failure

In some embodiments, the use of an antibody that specifically binds to N-terminal fragment of IGFBP-4 in the preparation of a reagent for diagnosing or assisting in the diagnosis of heart failure is provided.

In some embodiments, the antibody that specifically binds to N-terminal fragment of IGFBP-4 is used alone in the preparation of the reagent for diagnosing or assisting in the diagnosis of heart failure. In some embodiments, the antibody that specifically binds to N-terminal fragment of IGFBP-4 is used in combination with a reagent for detecting NT-proBNP in the preparation of the reagent for diagnosing or assisting in the diagnosis of heart failure.

In some embodiments, the use includes: using the antibody that specifically binds to the N-terminal fragment of insulin-like growth factor binding protein-4 to perform immunoassay to obtain the assay result of the N-terminal fragment of insulin-like growth factor binding protein-4; obtaining the assay result of an N-terminal pro-brain natriuretic peptide, and combining the assay result of the N-terminal pro-brain natriuretic peptide with the assay result of the N-terminal fragment of insulin-like growth factor binding protein-4 obtained by using the antibody for use in diagnosing or assisting in the diagnosis of heart failure.

In some embodiments, the diagnosing or assisting in the diagnosis of heart failure is either (i) or (ii) below:
(i) diagnosing or assisting in the diagnosis of whether a subject has heart failure;
(ii) diagnosing or assisting in the diagnosis of the severity of heart failure in a subject.

In some embodiments, the severity refers to left ventricular ejection fraction (LVEF%) or NYHA cardiac function classification. LVEF refers to the percentage of stroke volume in the ventricular end-diastolic solvent volume, is used to reflect left ventricular systolic function, and is one of important indicators for clinical judgment the severity of heart failure disease. The NYHA cardiac function classification is proposed by the New York Heart Disease Association, which contains a total of four classes, I-IV. NYHA cardiac function classification is a common clinical method for assessing the degree of cardiac function impairment, and is commonly used clinically to reflect a severity of the condition in heart failure patients. Among these, NYHA class I has the mildest degree, representing that the patient has heart disease but physical activity is not limited; NYHA class IV has the most severe degree, representing that the patient has heart disease, has symptoms of cardiac insufficiency or angina at rest, and any physical activity increases discomfort.

In some embodiments, a method for assessing whether a subject has heart failure is provided, which method includes:
detecting the level of N-terminal fragment of IGFBP-4 in a sample from the subject;
comparing the level of the N-terminal fragment of IGFBP-4 with a reference value, where
compared with the reference value, the level of N-terminal fragment of IGFBP-4 higher than the reference value indicates that the subject has heart failure;
compared with the reference value, the level of an N-terminal fragment of IGFBP-4 lower than the reference value indicates that the subject does not have heart failure.

In some embodiments, the method for assessing whether a subject has heart failure includes the following steps:
providing a reagent, where the reagent includes a magnetic solid phase carrier coated with a capture antibody, and an alkaline phosphatase-labelled detection antibody, where at least one of the capture antibody and the detection antibody specifically binds to the N-terminal fragment of IGFBP-4;
providing a sample from the subject;
mixing the sample with the magnetic solid phase carrier coated with the capture antibody and the alkaline phosphatase-labelled detection antibody, and incubating the mixture to obtain an immune complex;
providing a magnetic field condition, and washing the immune complex to remove unbound substances;
adding a chemiluminescent substrate to the washed immune complex, where the chemiluminescent substrate is suitable for being decomposed by an alkaline phosphatase to produce chemiluminescence; detecting an intensity of the chemiluminescence, and calculating the level of the N-terminal fragment of IGFBP-4.

In some embodiments, the method for assessing whether a subject has heart failure further includes:
detecting the level of NT-proBNP in a sample from the subject; based on the level of the N-terminal fragment of IGFBP-4 and the level of the NT-proBNP, determining whether the subject has heart failure or assessing the prognostic risk of heart failure in the subject.

In some embodiments, the level of the N-terminal fragment of IGFBP-4 and the level of the NT-proBNP are compared with their respective reference values, where
when the level of the NT-proBNP is lower than its reference value, it indicates that the subject does not have heart failure;
when the level of the N-terminal fragment of IGFBP-4 and the level of the NT-proBNP are both higher than their respective reference values, it indicates that the subject has heart failure.

### Product and method for assessing or assisting in the assessment of prognostic risk of heart failure

In some embodiments, the use of an antibody that specifically binds to N-terminal fragment of IGFBP-4 in the preparation of a reagent for assessing or assisting in the assessment of the prognostic risk of heart failure is provided.

In some embodiments, the antibody that specifically binds to N-terminal fragment of IGFBP-4 is used alone in the preparation of the product for assessing or assisting in the assessment of the prognostic risk of heart failure. In some embodiments, the antibody that specifically binds to N-terminal fragment of IGFBP-4 is used in combination with a reagent for detecting NT-proBNP in the preparation of the reagent for assessing or assisting in the assessment of the prognostic risk of heart failure.

In some embodiments, the use includes: using the antibody that specifically binds to the N-terminal fragment of insulin-like growth factor binding protein-4 to perform immunoassay to obtain the assay result of the N-terminal fragment of insulin-like growth factor binding protein-4; obtaining the assay result of an N-terminal pro-brain natriuretic peptide, and combining the assay result of the N-terminal pro-brain natriuretic peptide with the assay result of the N-terminal fragment of insulin-like growth factor binding protein-4 obtained by using the antibody for use in assessing or assisting in the assessment of the prognostic risk of heart failure.

In some embodiments, the assessing or assisting in the assessment of the prognostic risk of heart failure is either (iii) or (iv) below:
(iii) assessing or assisting in the assessment of the short-term prognostic risk in a heart failure patient, the short-term referring to a period less than 30 days;
(iv) assessing or assisting in the assessment of a long-term prognostic risk in a heart failure patient, the long-term referring to a period more than 30 days and not exceeding 240 days.

In some embodiments, the prognostic risk refers to the risk of cardiac-related rehospitalization and/or death.

In some embodiments, a method for assessing the prognostic risk of heart failure in a subject is provided, which method includes:
detecting the level of N-terminal fragment of IGFBP-4 in a sample from the subject;
comparing the level of the N-terminal fragment of IGFBP-4 with a reference value, where
compared with the reference value, the level of N-terminal fragment of IGFBP-4 higher than the reference value indicates that the subject has a high risk of poor prognosis for heart failure;
compared with the reference value, the level of N-terminal fragment of IGFBP-4 lower than the reference value indicates that the subject has a low risk of poor prognosis for heart failure.

In some embodiments, the method for assessing the prognostic risk of heart failure in a subject includes the following steps:
providing a reagent, where the reagent includes a magnetic solid phase carrier coated with a capture antibody, and an alkaline phosphatase-labelled detection antibody, where at least one of the capture antibody and the detection antibody is capable of specifically binding to the N-terminal fragment of IGFBP-4;
providing a sample from the subject;
mixing the sample with the magnetic solid phase carrier coated with the capture antibody and the alkaline phosphatase-labelled detection antibody, and incubating the mixture to obtain an immune complex;
providing a magnetic field condition, and washing the immune complex to remove unbound substances;
adding a chemiluminescent substrate to the washed immune complex, where the chemiluminescent substrate is suitable for being decomposed by an alkaline phosphatase to produce chemiluminescence; detecting an intensity of the chemiluminescence, and calculating the level of the N-terminal fragment of IGFBP-4.

In some embodiments, the method for assessing the prognostic risk of heart failure in a subject further includes:
detecting the level of NT-proBNP in a sample from the subject; based on the level of the N-terminal fragment of IGFBP-4 and the level of the NT-proBNP, determining whether the subject has heart failure or assessing the prognostic risk of heart failure in the subject.

In some embodiments, the level of the N-terminal fragment of IGFBP-4 and the level of the NT-proBNP are compared with their respective reference values, where
when the level of the N-terminal fragment of IGFBP-4 and the level of the NT-proBNP are both higher than their respective reference values, it indicates that the subject has a high risk of poor prognosis for heart failure;
when only one of the level of the N-terminal fragment of IGFBP-4 and the level of the NT-proBNP is higher than its respective reference value, it indicates that the subject has a moderate risk of poor prognosis for heart failure;
when the level of the N-terminal fragment of IGFBP-4 and the level of the NT-proBNP are both lower than their respective reference values, it indicates that the subject has a low risk of poor prognosis for heart failure.

### Product and method for assessing or assisting in the assessment of prognostic risk of coronary heart disease

In some embodiments, the use of an antibody that specifically binds to an N-terminal fragment of IGFBP-4 in the preparation of a reagent for assessing or assisting in the assessment of the prognostic risk of coronary heart disease is provided.

In some embodiments, the prognostic risk of coronary heart disease refers to the risk of cardiac-related rehospitalization and/or death.

In some embodiments, a method for assessing the prognostic risk of coronary heart disease is provided, which method includes:
detecting the level of N-terminal fragment of IGFBP-4 in a sample from a coronary heart disease patient;
comparing the level of the N-terminal fragment of IGFBP-4 with a reference value, where
compared with the reference value, the level of N-terminal fragment of IGFBP-4 higher than the reference value indicates that the subject has a high risk of poor prognosis for coronary heart disease;
compared with the reference value, the level of N-terminal fragment of IGFBP-4 lower than the reference value indicates that the subject has a low risk of poor prognosis for coronary heart disease.

### Kit

In some embodiments, a kit for detecting NT-IGFBP-4 is provided that can be used to detect NT-IGFBP-4 by double-antibody sandwich chemiluminescence immunoassay.

The kit includes:
a magnetic bead coating working solution, including a magnetic solid phase carrier (such as a mixture of superparamagnetic microparticles) coated with a capture antibody;
an enzyme-labelled substance working solution, including an alkaline phosphatase-labelled detection antibody.

At least one of the capture antibody and the detection antibody is an antibody that specifically binds to NT-IGFBP-4, and the capture antibody, the detection antibody, and NT-IGFBP-4 are capable of forming an immune complex. In some embodiments, an epitope recognized by the antibody that specifically binds to NT-IGFBP-4 includes a neoepitope formed at the N-terminal end of IGFBP-4 after cleavage of full-length IGFBP-4 by pregnancy-associated plasma protein-A (PAPP-A), where the full-length IGFBP-4 does not include the neoepitope structure, and therefore it cannot be recognized by the antibody.

In some embodiments, one of the capture antibody and the detection antibody is an antibody that specifically binds to NT-IGFBP-4, and the other is capable of recognizing and binding to NT-IGFBP-4 or full-length IGFBP-4.

The above capture antibody and detection antibody are commercially available, such as the monoclonal antibody available from Hytest under Cat.#4IGF4, specifically as follows:
monoclonal antibody IBP3cc (4IGF4-IBP3cc), which specifically binds to human NT-IGFBP-4, with a binding rate to full-length human IGFBP-4 of < 5% (ELISA); other antibodies that are capable of specifically binding to the neoepitope structure formed at the N-terminal end of IGFBP-4 after cleavage by PAPP-A are also suitable for use in the present disclosure.

Monoclonal antibodies IBP144, IBP154, and IBP180 (4IGF4-1BP144, 4IGF4-IBP154, and 4IGF4-IBP180) are capable of binding to both human IGFBP-4 and human NT-IGFBP-4. The epitopes recognized by these antibodies are located within positions 1-156 of the amino acid sequence of IGFBP-4, and other antibodies that are capable of recognizing the epitopes located within positions 1-156 of the amino acid sequence of IGFBP-4 are also suitable for use in the present disclosure.

In some embodiments, the capture antibody may be monoclonal antibody IBP3cc, and the detection antibody may be selected from monoclonal antibodies IBP144, IBP154, and IBP180. In other embodiments, the capture antibody may be selected from monoclonal antibodies IBP144, IBP154, and IBP180, and the detection antibody may be monoclonal antibody IBP3cc. Preferably, the capture antibody is an antibody that specifically binds to human NT-IGFBP-4.

In some embodiments, detection of human NT-IGFBP-4 is performed by Sandwich Immunoassay, the combination of the capture antibody and the detection antibody may be selected from any one of the following groups:
IBP3cc-IBP144;
IBP3cc-IBP180;
IBP3cc-IBP154.

The above kit can be used in combination with Mindray fully automated chemiluminescence immunoassay analysers such as CL2000i, CL6000i, and CL8000i. The principle and procedure for the NT-IGFBP-4 detection are as follows:
Step 1: A sample, a magnetic bead coating working solution, and an enzyme-labelled substance working solution are added to a reaction tube. After incubation, NT-IGFBP-4 in the sample binds to the capture antibody coated on the magnetic bead, while the detection antibody-alkaline phosphatase conjugate binds to NT-IGFBP-4 in the sample. After the reaction is complete, the solid phase is placed in a magnetic field. The magnetic field attracts the magnetic beads. The substance bound to the solid phase is retained, and the unbound substances are washed away.
Step 2: Chemiluminescent substrate (3-(2-spiroadamantane)-4-methoxy-4-(3-phosphoryloxy)-phenyl-1,2-dioxetane, AMPPD) is added to the reaction tube, and decomposed by alkaline phosphatase, and phosphate group is removed to produce an unstable intermediate. The intermediate generates a methyl m-oxybenzoate anion by intramolecular electron transfer, and the chemiluminescence is generated as the methyl m-oxybenzoate anion in the excited state returning to the ground state. The number of photons generated during the reaction is then measured using a photomultiplier tube. The number of photons generated is proportional to the concentration of NT-IGFBP-4 in the sample. The amount of analyte in the sample is determined by the calibration curve.

### Exemplary embodiments

Specific embodiments of the present invention further include:
Embodiment 1. Use of an antibody that specifically binds to N-terminal fragment of insulin-like growth factor binding protein-4 in the preparation of a reagent for clinical assessment of heart failure, wherein
   the N-terminal fragment of insulin-like growth factor binding protein-4 is the N-terminal fragment obtained by catalytic hydrolysis of insulin-like growth factor binding protein-4 by pregnancy-associated plasma protein A;
   the reagent for clinical assessment of heart failure is used for diagnosing or assisting in the diagnosis of heart failure, or for assessing or assisting in the assessment of prognostic risk of heart failure.
Embodiment 2. The use of Embodiment 1, wherein the N-terminal fragment of insulin-like growth factor binding protein-4 comprises the sequence as set forth in SEQ ID NO: 1.
Embodiment 3. The use of Embodiment 1, wherein the use further comprises: using the antibody that specifically binds to the N-terminal fragment of insulin-like growth factor binding protein-4 to perform immunoassay to obtain a detection result of the N-terminal fragment of insulin-like growth factor binding protein-4; obtaining a detection result of N-terminal pro-brain natriuretic peptide, and combining the detection result of the N-terminal pro-brain natriuretic peptide with the detection result of the N-terminal fragment of insulin-like growth factor binding protein-4 for use in diagnosing or assisting in the diagnosis of heart failure, or assessing or assisting in the assessment of prognostic risk of heart failure.
Embodiment 4. The use of any one of Embodiments 1 to 3, wherein the diagnosing or assisting in the diagnosis of heart failure is either (i) or (ii) below:
   (i) diagnosing or assisting in the diagnosis of whether a subject has heart failure;
   (ii) diagnosing or assisting in the diagnosis of a severity of heart failure in a subject;
   preferably, the severity refers to left ventricular ejection fraction or NYHA cardiac function classification.
Embodiment 5. The use of any one of Embodiments 1 to 3, wherein the assessing or assisting in the assessment of prognostic risk of heart failure is either (iii) or (iv) below:
   (iii) assessing or assisting in the assessment of short-term prognostic risk in a heart failure patient, the short-term referring to a period less than 30 days;
   (iv) assessing or assisting in the assessment of long-term prognostic risk in a heart failure patient, the long-term referring to a period more than 30 days and less than 240 days;
   preferably, the prognostic risk refers to the risk of cardiac-related rehospitalization and/or death.
Embodiment 6. Use of an antibody that specifically binds to N-terminal fragment of insulin-like growth factor binding protein-4 in the preparation of a reagent for clinical assessment of coronary heart disease, wherein
   the N-terminal fragment of insulin-like growth factor binding protein-4 is: an N-terminal fragment obtained by catalytic hydrolysis of insulin-like growth factor binding protein-4 by pregnancy-associated plasma protein A;
   the reagent for clinical assessment of coronary heart disease is used for assessing or assisting in the assessment of prognostic risk of coronary heart disease.
Embodiment 7. The use of Embodiment 6, wherein the N-terminal fragment of insulin-like growth factor binding protein-4 comprises the sequence as set forth in SEQ ID NO: 1.
Embodiment 8. The use of Embodiment 6, wherein the prognostic risk of coronary heart disease refers to the risk of cardiac-related rehospitalization and/or death.
Embodiment 9. A method for assessing whether a subject has heart failure or assessing prognostic risk of heart failure in a subject, wherein the method comprises:
   detecting a level of N-terminal fragment of insulin-like growth factor binding protein-4 in a sample from the subject, wherein the N-terminal fragment of insulin-like growth factor binding protein-4 is an N-terminal fragment obtained by catalytic hydrolysis of insulin-like growth factor binding protein-4 by pregnancy-associated plasma protein A;
   comparing the level of the N-terminal fragment of insulin-like growth factor binding protein-4 with a reference value, wherein
   compared with the reference value, a level of the N-terminal fragment of insulin-like growth factor binding protein-4 higher than the reference value indicates that the subject has heart failure or has a high risk of poor prognosis for heart failure;
   compared with the reference value, a level of the N-terminal fragment of insulin-like growth factor binding protein-4 lower than the reference value indicates that the subject does not have heart failure or has a low risk of poor prognosis for heart failure.
Embodiment 10. The method of Embodiment 9, wherein detecting the level of the N-terminal fragment of insulin-like growth factor binding protein-4 in a sample from the subject comprises the following steps:
   providing a sample from the subject;
   providing a reagent comprising an antibody that specifically binds to the N-terminal fragment of insulin-like growth factor binding protein-4; using the reagent to perform immunoassay on the sample to obtain a detection result of the level of the N-terminal fragment of insulin-like growth factor binding protein-4.
Embodiment 11. The method of Embodiment 10, wherein detecting the level of the N-terminal fragment of insulin-like growth factor binding protein-4 in a sample from the subject comprises the following steps:
   providing a reagent, wherein the reagent comprises a magnetic solid phase carrier coated with a capture antibody, and an alkaline phosphatase-labelled detection antibody, wherein at least one of the capture antibody and the detection antibody is capable of specifically binding to the N-terminal fragment of insulin-like growth factor binding protein-4;
   providing a sample from the subject;
   mixing the sample with the magnetic solid phase carrier coated with the capture antibody and the alkaline phosphatase-labelled detection antibody, and incubating the mixture to form an immune complex;
   providing a magnetic field condition, and washing the immune complex to remove unbound substances;
   adding a chemiluminescent substrate to the washed immune complex, wherein the chemiluminescent substrate is suitable for being decomposed by alkaline phosphatase to produce chemiluminescence; detecting intensity of the chemiluminescence, and calculating the level of the N-terminal fragment of insulin-like growth factor binding protein-4.
Embodiment 12. The method of any one of Embodiments 9 to 11, wherein the method further comprises:
   detecting a level of N-terminal pro-brain natriuretic peptide in a sample from the subject;
   based on the level of the N-terminal fragment of insulin-like growth factor binding protein-4 and the level of the N-terminal pro-brain natriuretic peptide, determining whether the subject has heart failure or assessing the prognostic risk of heart failure in the subject.
Embodiment 13.A method for assessing prognostic risk of coronary heart disease, wherein the method comprises:
   detecting a level of N-terminal fragment of insulin-like growth factor binding protein-4 in a sample from a coronary heart disease patient, wherein the N-terminal fragment of insulin-like growth factor binding protein-4 is the N-terminal fragment obtained by catalytic hydrolysis of insulin-like growth factor binding protein-4 by pregnancy-associated plasma protein A;
   comparing the level of the N-terminal fragment of insulin-like growth factor binding protein-4 with a reference value, wherein
   compared with the reference value, a level of the N-terminal fragment of insulin-like growth factor binding protein-4 higher than the reference value indicates that the subject has a high risk of poor prognosis for coronary heart disease;
   compared with the reference value, a level of the N-terminal fragment of insulin-like growth factor binding protein-4 lower than the reference value indicates that the subject has a low risk of poor prognosis for coronary heart disease.
Embodiment 14. The method of Embodiment 13, wherein the N-terminal fragment of insulin-like growth factor binding protein-4 comprises the sequence as set forth in SEQ ID NO: 1.

Examples of the present disclosure will be enumerated hereinafter, and advantages and various effects of the present disclosure will be presented more clearly therefrom. It will be understood by those skilled in the art that these examples are used to illustrate the present disclosure and not to limit the present disclosure.

### EXAMPLE 1

A kit for detecting NT-IGFBP-4 was provided. Using the kit, NT-IGFBP-4 was detected by double-antibody sandwich chemiluminescence immunoassay.

The kit included:
a magnetic bead coating working solution, including a mixture of superparamagnetic microparticles coated with capture antibody, where the magnetic bead coating working solution was used to capture NT-IGFBP-4 in a sample;
an enzyme-labelled substance working solution, including alkaline phosphatase-labelled detection antibody, where the enzyme-labelled substance working solution was used to detect an NT-IGFBP-4 antigen captured by the superparamagnetic particles.

The capture antibody was monoclonal antibody IBP3cc, and the detection antibody was monoclonal antibody IBP144.

The above kit was used in combination with Mindray fully automated chemiluminescence immunoassay analyser CL80001 for the detection of NT-IGFBP-4 in subsequent examples.

### EXAMPLE 2

This example analysed the difference in the level of NT-IGFBP-4 between an apparently healthy population and a heart failure population.

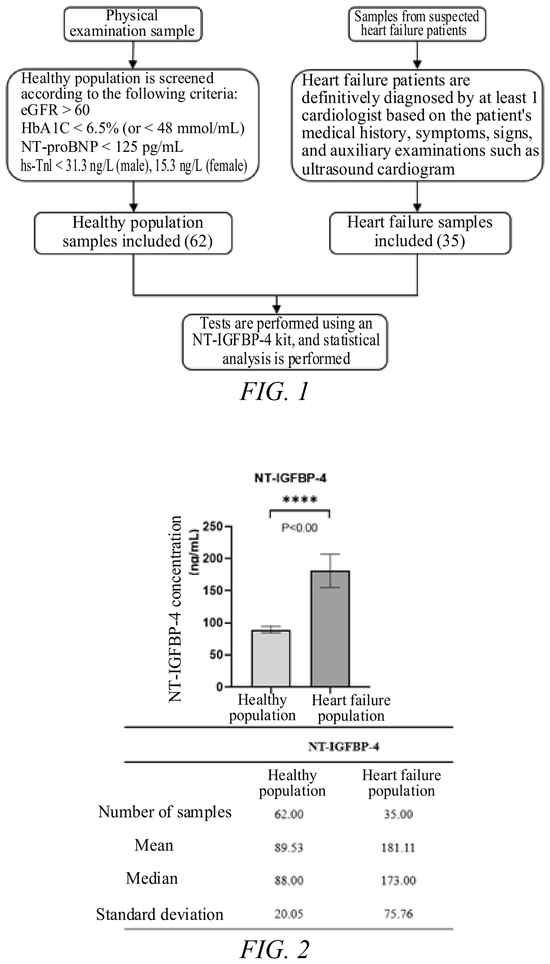

To evaluate the test application of the NT-IGFBP-4 detection kit under conventional clinical conditions, the concentration levels of NT-IGFBP-4 in plasma samples from a population of patients with confirmed heart failure (n = 35) and an apparently healthy population (n = 62) were separately investigated. The apparently healthy population was further screened from a self-reported healthy adult population undergoing physical examinations based on exclusion criteria including eGFR > 60, HbA1C (glycated haemoglobin)< 6.5% (or < 48 mmol/mL), NT-proBNP < 125 pg/mL, and hs-TnI < 31.3 ng/L (male) or 15.3 ng/L (female). The population of heart failure patients was definitively diagnosed by at least 1 cardiologist based on the patient's medical history, symptoms, signs, and auxiliary examinations such as ultrasound cardiogram. The inclusion process for the samples from the two populations was as shown in FIG. 1. Blood samples were collected from both populations upon admission using heparin lithium vacuum blood collection tubes (BD, CNL20-C0371). After processing, the samples were tested using the NT-IGFBP-4 kit provided in Example 1. Statistical analysis was performed using Graph Pad Prism 9.0 software, comparisons between groups of measurement data were performed using the t-test (normal distribution), and two-tailed significance level of P < 0.05 was considered statistically significant.

The results are shown in FIG. 2. The difference in measured NT-IGFBP-4 concentrations between the apparently healthy population and the heart failure population was quite significant (statistical difference P < 0.0001). The average value of NT-IGFBP-4 in the healthy population was 89.53, while the average value in the heart failure population significantly increased to 181.11, with a significant correlation between NT-IGFBP-4 protein concentration and heart failure disease, indicating that NT-IGFBP-4 may have important application value in the diagnosis and prognosis of heart failure.

### EXAMPLE 3

This example was used to evaluate the application value of NT-IGFBP-4 in the diagnosis of heart failure.

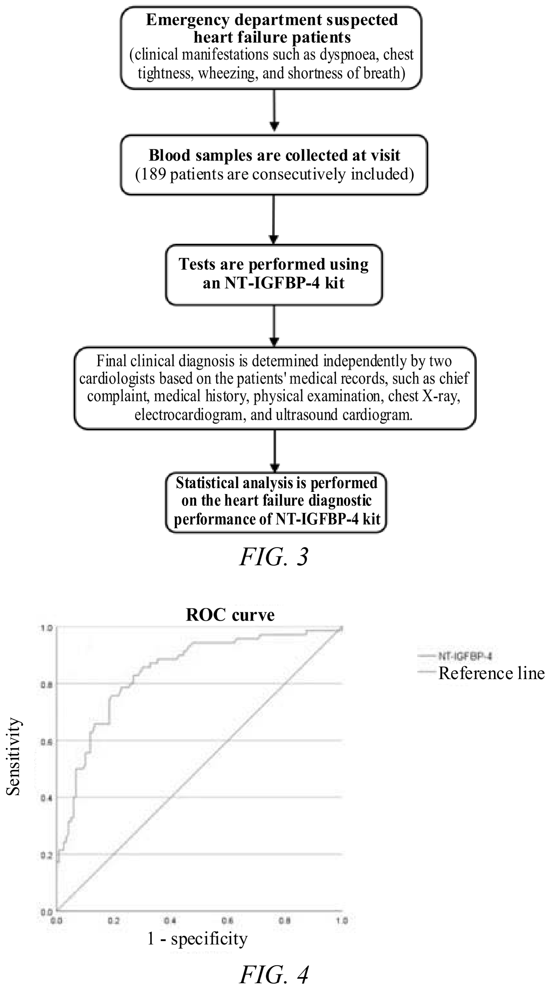

This example consecutively included 189 patients who presented to the hospital emergency department with clinical manifestations such as dyspnoea, chest tightness, wheezing, shortness of breath, and other suspected heart failure symptoms. The final clinical diagnosis was determined independently by two cardiologists based on the patients' medical records, such as chief complaint, medical history, physical examination, chest X-ray, electrocardiogram, and ultrasound cardiogram etc., of which 70 patients had a positive diagnosis of heart failure and 119 patients had a negative diagnosis of heart failure. The specific sample inclusion process is shown in FIG. 3.

Blood samples were collected on the day of the patients' hospital visit using heparin lithium vacuum blood collection tubes (BD, CNL20-C0371). After processing, the samples were tested using the NT-IGFBP-4 kit provided in Example 1. Statistical analysis was performed using IBM SPSS Statistics (27.0) to calculate the area under the receiver operating characteristic curve (ROC)(AUC), the sensitivity, the specificity, etc. Two-tailed significance level of P < 0.05 was considered statistically significant. The results are shown in FIG. 4 and Table 1. For the evaluation of the diagnostic value of NT-IGFBP-4 in heart failure, the area under the ROC curve (AUC) was 0.841. When the Youden index was maximum at 0.564, the optimal diagnostic threshold was 148.5 ng/mL, with a sensitivity and specificity of 75.7% and 80.7%, respectively. The negative predictive value (85.0%) was more significant than the positive predictive value (69.7%).

**Table 1**

| Marker | ROC_ AUC | Youden index | Determina tion threshold/ ng/mL | Sensitivity | Specificity | Negative predictive value (NPV) | Positive predictive value (PPV) |
|---|---|---|---|---|---|---|---|
| NT-IGFBP-4 | 0.841 | 0.564 | > 148.5 | 75.7% | 80.7% | 85.0% | 69.7% |

### EXAMPLE 4

This example was used to evaluate the application value of combination of NT-IGFBP-4 and NT-proBNP in the diagnosis of heart failure.

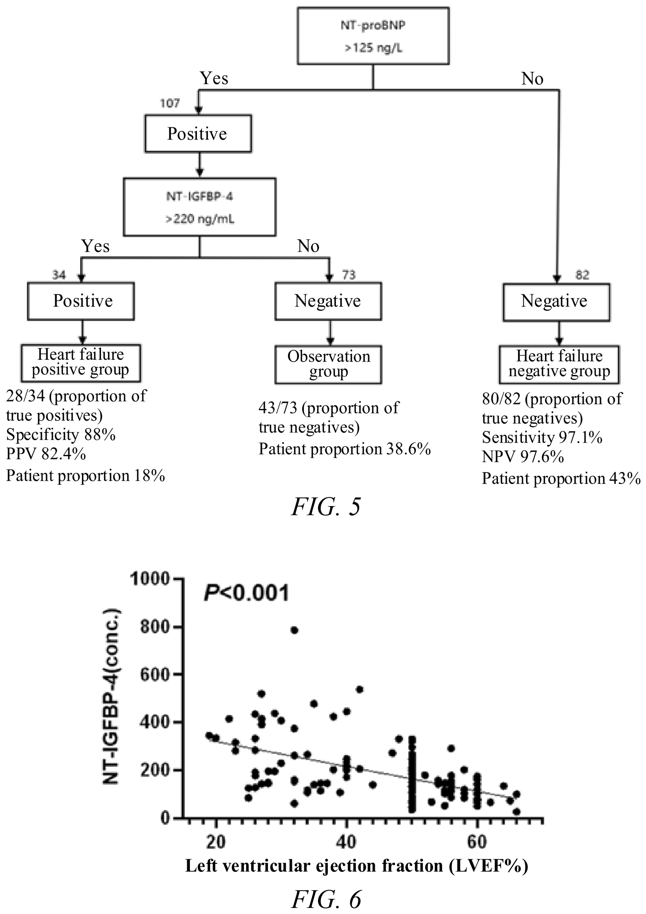

On the basis of Example 3, NT-proBNP in samples from the 189 patients were detected (Mindray, #105-027045-00), and the diagnostic value for heart failure of using NT-proBNP alone, using NT-IGFBP-4 alone, and using a combination of NT-IGFBP-4 and NT-proBNP were analysed. In the case of combined diagnosis using a combination of NT-IGFBP-4 and NT-proBNP, the positive or negative diagnosis of heart failure was determined according to the combined diagnosis decision tree of FIG. 5.

The patients were divided into an NT-proBNP negative group and an NT-proBNP positive group according to the guideline threshold for NT-proBNP, where the NT-proBNP negative group was the heart failure negative group. In NT-proBNP positive group, the diagnosis threshold of NT-IGFBP-4 was combined to grouping, among which NT-IGFBP-4 positive group was heart failure positive group, NT-IGFBP-4 negative group was observation group, which should be confirmed in combination with other clinical indicators such as ultrasound cardiogram. The diagnostic threshold of NT-IGFBP-4 (220 ng/mL) was derived based on the criterion that the specificity should be at least 85%. The application performance results of the diagnostic decision tree of using a combination of NT-IGFBP-4 and NT-proBNP are shown in Table 2 and FIG. 5.

**Table 2**

| Marker | Determination threshold | Sensitivity | Specificity | NPV | PPV |
|---|---|---|---|---|---|
| NT-proBNP | > 125 ng/L | 97.1% | 67.2% | 97.6% | 63.6% |
| Combination of NT-IGFBP-4 and NT-proBNP | > 220 ng/mL | 97.1% | 88% | 97.6% | 82.4% |
| | > 125 ng/L | | | | |

Referring to Table 2 and FIG. 5, when a combination of NT-IGFBP-4 and NT-proBNP was used for diagnosis, the specificity was significantly increased from 67.2% to 88% while maintaining high sensitivity, and the positive predictive value (PPV) was increased from 63.6% to 82.4%. This indicates that when the measured NT-proBNP is lower than the diagnostic threshold in clinical practice, the test patient has a 97.6% probability of being negative for heart failure and can be safely ruled out; when both measured values are higher than the diagnostic thresholds, the test patient has an 82.4% probability of being positive for heart failure and clinical treatment should be considered; when NT-proBNP is positive and NT-IGFBP-4 is negative, the patient requires observation combined with other clinical means.

In this example, the sensitivity of NT-proBNP for ruling out heart failure reaches 97.1%, but its specificity when used alone is low, only 67.2%. By means of combined diagnosis with NT-IGFBP-4, the diagnostic specificity and positive predictive value (PPV) are significantly improved.

NT-proBNP, as a commonly used marker of heart failure in clinical practice at present, has great application value in negative exclusion of heart failure due to its high sensitivity, but due to its very low specificity (according to this example, only 67.2%) and PPV, in clinical practice, NT-proBNP-positive patients often require confirmation through a combination of multiple other clinical detection methods such as ultrasound cardiogram, electrocardiogram. The use of a combination of NT-IGFBP-4 and NT-proBNP for diagnosis results in a significant increase in diagnostic specificity and PPV. This allows about 1/3 of NT-proBNP-positive patients to be considered for treatment without the need for multiple other clinical tests, which is beneficial for reducing medical costs, avoiding resource occupancy, and improving hospital turnover rates, etc.

In summary, through the combined use of NT-IGFBP-4 and NT-proBNP, patients presenting for clinical care are classified into three categories: those who are negative for heart failure and can be safely ruled out, those who are positive for heart failure and can be considered for treatment, and those under observation who require other examinations for final confirmation. Compared with the use of NT-proBNP alone, the use of the combination significantly improves the specificity and PPV of heart failure diagnosis while ensuring high sensitivity and NPV, thereby providing good benefits for clinical patients.

### EXAMPLE 5

Both left ventricular ejection fraction (LVEF) and NYHA cardiac function classification score are indicators for evaluating the severity of heart failure. This example evaluated the correlation of NT-IGFBP-4 with the left ventricular ejection fraction (LVEF) and the NYHA cardiac function classification score. Based on the evaluation results, the measured NT-IGFBP-4 can be used to diagnose or assist in the diagnosis of the severity of the condition in heart failure patients.

### I. Left ventricular ejection fraction (LVEF)

LVEF refers to the percentage of stroke volume in the ventricular end-diastolic solvent volume, is used to reflect left ventricular systolic function, and is one of important indicators for clinical judgment of heart failure disease. In general, the normal value of LVEF should be ≥ 50%, and a value < 50% suggests the presence of signs of cardiac insufficiency in the patient.

136 patients from the cardiology department with available LVEF values during the current outpatient or emergency visits were consecutively and randomly included. Blood samples were collected on the day of the patients' hospital visit using heparin lithium vacuum blood collection tubes (BD, CNL20-C0371). After processing, the samples were tested using the NT-IGFBP-4 kit provided in Example 1. Statistical analysis was performed using Graph Pad Prism 9.0, and two-tailed significance level of P < 0.05 was considered statistically significant.

As shown in FIG. 6, the concentration level of NT-IGFBP-4 exhibited a statistically significant correlation with left ventricular ejection function (significance level P < 0.001). The higher the measured concentration of NT-IGFBP-4, the lower the patient's left ventricular ejection fraction (LVEF%), representing a more severe degree of cardiac insufficiency in the patient. The above indicates that the concentration level of NT-IGFBP-4 has a significant correlation with left ventricular systolic function, and that the measured value reflects the severity of heart failure in the subject.

### II. NYHA cardiac function classification

The NYHA cardiac function classification is proposed by the New York Heart Disease Association. NYHA cardiac function classification contains a total of four classes, I-IV, and is a common clinical method for assessing the degree of cardiac function impairment, and is commonly used clinically to reflect the severity of the condition in heart failure patients. Among these, NYHA class I has the mildest degree, representing that the patient has heart disease but physical activity is not limited; NYHA class IV has the most severe degree, representing that the patient has heart disease, has symptoms of cardiac insufficiency or angina at rest, and any physical activity increases discomfort.

A total of 252 heart failure patients with a definite NYHA cardiac function classification during the current outpatient or emergency visits were consecutively and randomly included. The classification was determined by hospital clinicians based on the patients' overall clinical signs. Of these, there were 18 patients with NYHA class I, 69 patients with NYHA class II, 88 patients with NYHA class III, and 77 patients with NYHA class IV. Blood samples were collected on the day of the patients' hospital visit using heparin lithium vacuum blood collection tubes (BD, CNL20-C0371). After processing, the samples were tested using the NT-IGFBP-4 kit provided in Example 1. Statistical analysis was performed using Graph Pad Prism 9.0, and two-tailed significance level of P < 0.05 was considered statistically significant.

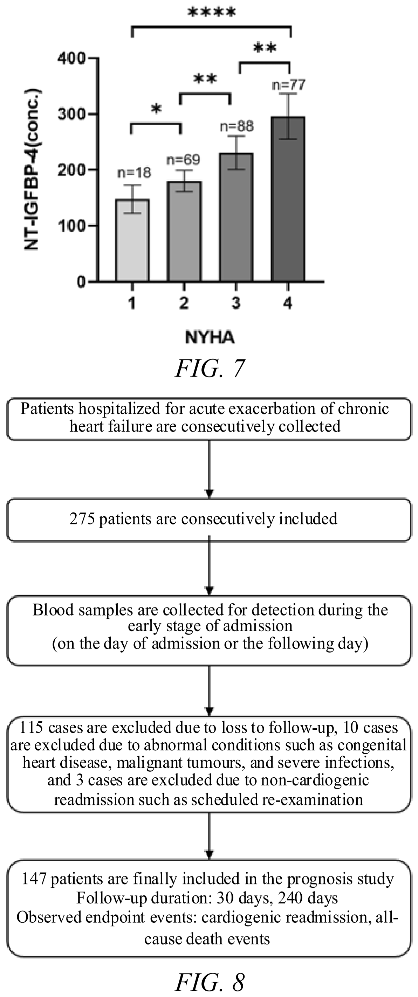

As shown in FIG. 7, for the measured concentration of NT-IGFBP-4 among the four groups (NYHA classes I, II, III, and IV), there were statistically significant differences between every two groups. The higher the measured concentration of NT-IGFBP-4, the higher the patient's NYHA cardiac function classification score, representing a more severe degree of the condition in the patient. The above indicates that the concentration level of NT-IGFBP-4 has a significant correlation with the NYHA cardiac function classification, and that the measured value is used to diagnose or assist in the diagnosis of the severity of the condition in heart failure patients.

### EXAMPLE 6

This example was used to evaluate the predictive value of NT-IGFBP-4 in the prognosis of heart failure.

In this example, 275 patients hospitalized for acute exacerbation of chronic heart failure were consecutively included. The follow-up lasted for 240 days, during which 115 patients were excluded due to loss during follow-up, 10 patients were excluded due to abnormal conditions such as congenital heart disease, malignant tumours, and severe infections, and an additional 3 patients were excluded due to non-cardiac-related rehospitalization during the follow-up. Finally, 147 patients were included in the prognosis study for statistical analysis. The specific process of inclusion of prognosis study samples is shown in FIG. 8. Fresh blood samples were collected from the patients during the early stage of admission (on the day of admission or the following day) using heparin lithium vacuum blood collection tubes (BD, CNL20-C0371). After processing, the samples were tested using the NT-IGFBP-4 kit provided in Example 1.

### I. Long-term prognosis of heart failure

Among the above 147 patients, a total of 32 patients experienced endpoint events within the 240-day follow-up duration, including 30 cases of cardiac-related rehospitalization and 2 cases of all-cause death. Based on the occurrence of endpoint events, the patients were divided into a survivor group and a non-survivor group.

The measured NT-IGFBP-4, the measured NT-proBNP, and a combination of the two were used for prognostic assessment within 240 days for heart failure. The efficacy for the prediction of prognostic risk within 240 days in the heart failure population of using NT-IGFBP-4 alone, using NT-proBNP alone, and using a combination of the two was analysed using ROC curves. For the use of a combination of the two, with the prognostic outcome as the dependent variable and the measured values of the two markers as the independent variables, logistic regression was constructed to calculate the predicted probability corresponding to each subject. The predicted probabilities were used for ROC analysis, and the AUC value was calculated. The results are shown in Table 3.

**Table 3**

| Test variable | AUC | Asymptotic significance | Asymptotic 95% confidence interval | | Optimal threshold |
|---|---|---|---|---|---|
| | | | Lower limit | Upper limit | |
| NT-proBNP | 0.730 | 0.000 | 0.630 | 0.831 | 3111 ng/L |
| NT-IGFBP-4 | 0.783 | 0.000 | 0.685 | 0.881 | 283 ng/mL |
| NT-proBNP+ NT-IGFBP-4 | 0.780 | 0.000 | 0.683 | 0.878 | / |

Based on the above results, the AUC of NT-IGFBP-4 for assessing long-term (240-day) prognostic risk of heart failure was 0.783, which was superior to that of NT-proBNP (AUC = 0.730) commonly used in clinical practice for heart failure, showing a statistically significant difference. At the highest Youden index, the optimal threshold of NT-IGFBP-4 was 283 ng/mL, and the optimal threshold of NT-proBNP was 3111 ng/L.

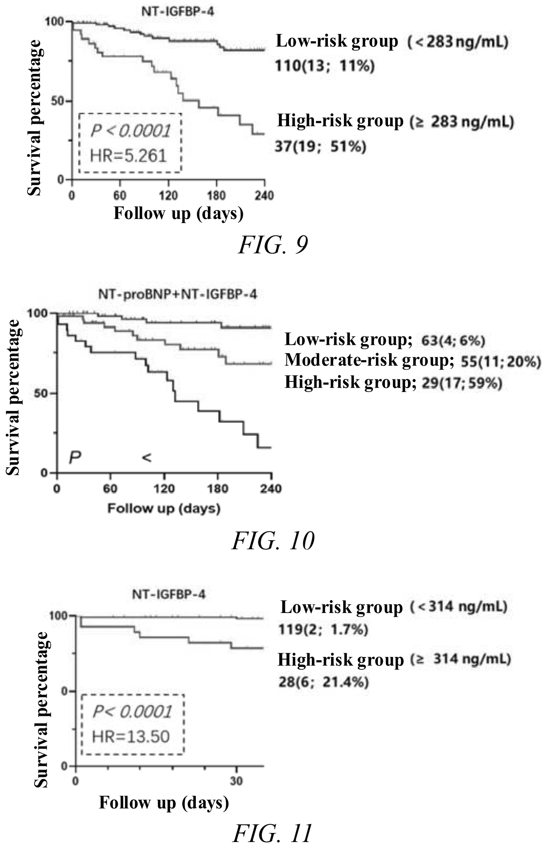

For a condition that the prognostic risk of heart failure was assessed using NT-IGFBP-4 alone, Kaplan-Meier survival curves of heart failure populations within 240-day follow-up duration are shown in FIG. 9. After dividing the population into risk groups using the optimal threshold of 283 ng/mL for NT-IGFBP-4, the high-risk group and the low-risk group showed a significant difference (P < 0.0001), with a hazard ratio (logrank) of 5.261 (95% Cl, 2.271-12.19). In the high-risk group, 51% of heart failure patients experienced an endpoint event within 240-day follow-up duration.

For a condition that the prognostic risk of heart failure was assessed using a combination of NT-IGFBP-4 and NT-proBNP, Kaplan-Meier survival curves of heart failure populations within 240-day follow-up duration are shown in FIG. 10. Heart failure patients were classified into high-risk, moderate-risk, and low-risk groups based on the thresholds of NT-IGFBP-4 and NT-proBNP, where patients with both markers higher than the thresholds were classified as the high-risk group, those with only one marker higher than the threshold were classified as the moderate-risk group, and those with both markers lower than the thresholds were classified as the low-risk group. As shown in FIG. 10, when a combination of NT-IGFBP-4 and NT-proBNP was used, the ability to stratify the prognostic risk of heart failure patients was more significant. In the high-risk group, 59% of heart failure patients experienced an endpoint event within 240-day follow-up duration .

The above analysis results show that both using NT-IGFBP-4 alone and using a combination of NT-IGFBP-4 and NT-proBNP are used to assess or assist in the assessment of the long-term (within 240 days) prognostic risk of heart failure, demonstrating good prognostic value. Furthermore, when a combination of NT-IGFBP-4 and NT-proBNP is used, the ability to stratify the prognostic risk of heart failure patients is more significant.

### II. Short-term prognosis of heart failure

Among the above 147 patients, a total of 8 patients experienced endpoint events within the 30-day follow-up duration, including 6 cases of cardiac-related rehospitalization and 2 cases of all-cause death. Based on the occurrence of endpoint events, the patients were divided into a survivor group and a non-survivor group.

The measured NT-IGFBP-4, the measured NT-proBNP, and a combination of the two were used for prognostic assessment within 30 days for heart failure. The efficacy for the prediction of prognostic risk within 30 days in the heart failure population of using NT-IGFBP-4 alone, using NT-proBNP alone, and using a combination of the two was analysed using ROC curves. For the use of a combination of the two, with the prognostic outcome as the dependent variable and the measured values of the two markers as the independent variables, logistic regression was constructed to calculate the predicted probability corresponding to each subject. The predicted probabilities were used for ROC analysis, and the AUC value was calculated. The results are shown in Table 4.

**Table 4**

| Test variable | AUC | Asymptotic significance | Asymptotic 95% confidence interval | | Optimal threshold |
|---|---|---|---|---|---|
| | | | Lower limit | Upper limit | |
| NT-proBNP | 0.669 | 0.108 | 0.499 | 0.839 | 3110 ng/L |
| NT-IGFBP-4 | 0.806 | 0.004 | 0.653 | 0.960 | 314 ng/mL |
| NT-proBNP+ NT-IGFBP-4 | 0.798 | 0.005 | 0.644 | 0.951 | / |

Based on the above results, the AUC of NT-IGFBP-4 for assessing short-term (30-day) prognostic risk of heart failure was 0.806, which was significantly higher than that of NT-proBNP (AUC = 0.669) commonly used in clinical practice for heart failure, showing a statistically significant difference. At the highest Youden index, the optimal threshold of NT-IGFBP-4 was 314 ng/mL, and the optimal threshold of NT-proBNP was 3110 ng/L.

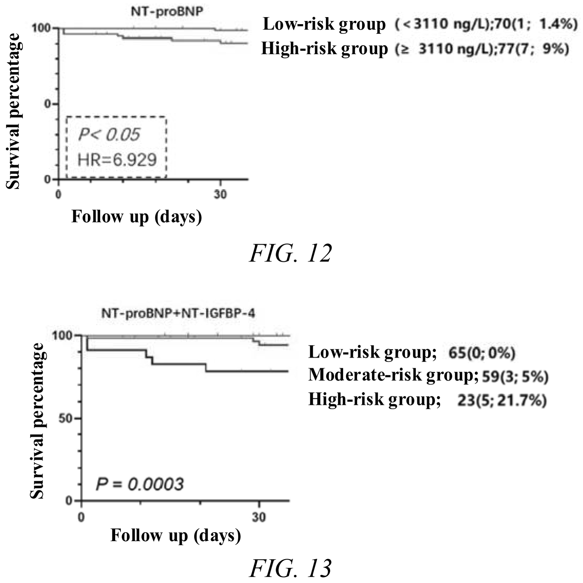

For conditions that the prognostic risk of heart failure was assessed using NT-IGFBP-4 alone and using NT-proBNP alone, Kaplan-Meier survival curves of heart failure populations within 30-day follow-up duration are shown in FIG. 11 and FIG. 12, respectively. After dividing the population into risk groups using the optimal threshold of 314 ng/mL for NT-IGFBP-4, the high-risk group and the low-risk group showed a significant difference (P < 0.0001). In the high-risk group, 21.4% of heart failure patients experienced an endpoint event within 30 days, with a hazard ratio (logrank) of 13.50 (95% Cl, 2.239-81.38), while the hazard ratio of NT-proBNP was only 6.929 (95%, 1.733-27.70, P < 0.05). During the 30-day short-term follow-up of heart failure patients, the prognostic value of NT-IGFBP-4 was significant, and the prognostic effect was significantly superior to that of NT-proBNP currently commonly used in clinical practice for heart failure.

For a condition that the prognostic risk of heart failure was assessed using a combination of NT-IGFBP-4 and NT-proBNP, Kaplan-Meier survival curves of heart failure populations within 30-day follow-up duration are shown in FIG. 13. Heart failure patients were classified into high-risk, moderate-risk, and low-risk groups based on the thresholds of NT-IGFBP-4 and NT-proBNP, where patients with both markers higher than the thresholds were classified as the high-risk group, those with only one marker higher than the threshold were classified as the moderate-risk group, and those with both markers lower than the thresholds were classified as the low-risk group. As shown in FIG. 13, when a combination of NT-IGFBP-4 and NT-proBNP was used, in the high-risk group, 21.7% of heart failure patients experienced an endpoint event within the 30-day prognostic period. The ability of the combination of the two to stratify the prognostic risk of heart failure patients was similar to the effect of using NT-IGFBP-4 alone, indicating that the efficacy of using NT-IGFBP-4 alone is already sufficient to predict a 30-day short-term prognosis of heart failure patients.

### EXAMPLE 7

This example was used to evaluate the predicted value of NT-IGFBP-4 in the prognosis of coronary heart disease.

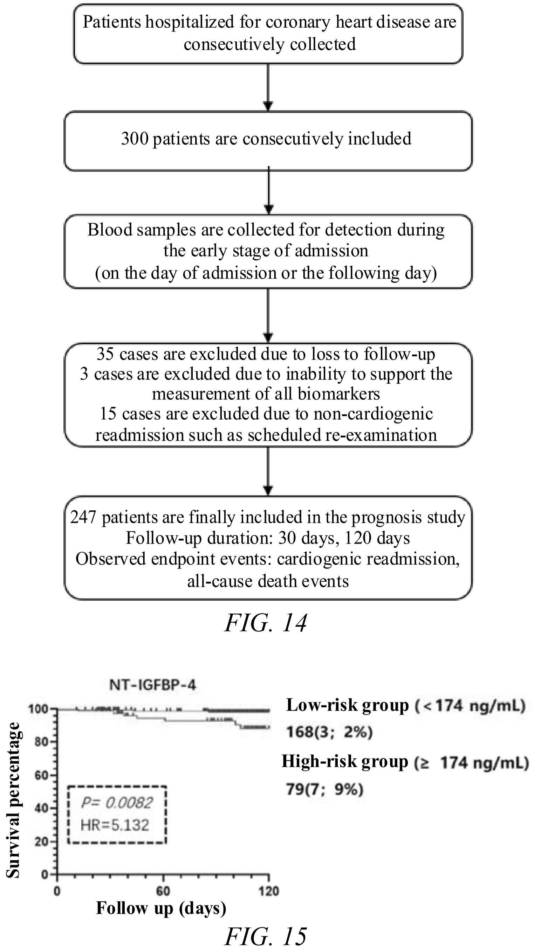

In this example, 300 patients hospitalized for coronary heart disease were consecutively included. The follow-up lasted for 120 days, during which 35 patients were excluded due to loss during follow-up, 3 patients were excluded due to the inability of the samples to support the measurement of all biomarkers, and an additional 15 patients were excluded due to non-cardiac-related rehospitalization such as scheduled re-examination during the follow-up. Finally, 247 patients were included in the prognosis study for statistical analysis. The specific process for including prognosis study samples is shown in FIG. 14. Fresh blood samples were collected from the patients during the early stage of admission (on the day of admission or the following day) using heparin lithium vacuum blood collection tubes (BD, CNL20-C0371). After processing, the samples were tested using the NT-IGFBP-4 kit provided in Example 1. Statistical analysis was performed using Graph Pad Prism 9.0 and IBM SPSS Statistics (27.0) software, and two-tailed significance level of P < 0.05 was considered statistically significant.

Among the above 247 patients, 13 patients experienced the observed endpoint events within 120 days, and 234 patients experienced no events. In this example, the value of NT-IGFBP-4 in assessing the 120-day prognostic risk in the coronary heart disease patient population was evaluated using ROC curve analysis. The results are shown in Table 5.

**Table 5**

| Test variable | AUC | Asymptotic significance | Asymptotic 95% confidence interval | | Optimal threshold |
|---|---|---|---|---|---|
| | | | Lower limit | Upper limit | |
| NT-IGFBP-4 | 0.708 | 0.026 | 0.540 | 0.875 | 174 ng/mL |

These results suggest that the AUC of NT-IGFBP-4 for assessing 120-day prognostic risk in patients with coronary heart disease was 0.708, with a statistically significant difference (asymptotic significance <0.05), and the optimal threshold was 174 ng/mL (highest Joden index).

Referring to FIG. 15, the Kaplan-Meier survival curves of coronary heart disease populations within the 120-day follow-up duration show that, after dividing the population into risk groups using the optimal threshold of 174 ng/mL for NT-IGFBP-4, the high-risk group (higher than the threshold) and the low-risk group (lower than the threshold) showed a significant difference (P < 0.05), with a hazard ratio (logrank) of 5.132 (95% Cl, 1.348-19.54). The above analysis results show that NT-IGFBP-4 can be used to assess or assist in the assessment of the prognostic risk of coronary heart disease, demonstrating good prognostic value.

The above descriptions are merely preferred embodiments of the present disclosure, but the scope of protection of the present disclosure is not limited thereto. Changes or substitutions readily figured out by those skilled in the art within the technical scope disclosed in the present disclosure shall fall within the scope of protection of the present disclosure. Therefore, the scope of protection of the present disclosure shall be subject to the scope of protection of the claims.

## Claims

1. A method for diagnosing or assisting in the diagnosis of heart failure or assessing or assisting in the assessment of prognostic risk of heart failure in a subject, wherein the method comprises:
detecting a level of N-terminal fragment of insulin-like growth factor binding protein-4 (IGFBP-4) in a sample from the subject, wherein the N-terminal fragment of insulin-like growth factor binding protein-4 is an N-terminal fragment obtained by catalytic hydrolysis of insulin-like growth factor binding protein-4 by pregnancy-associated plasma protein A;
diagnosing or assisting in the diagnosis of heart failure or assessing or assisting in the assessment of prognostic risk of heart failure based on the level of N-terminal fragment of insulin-like growth factor binding protein-4.

2. The method of claim 1, wherein diagnosing or assisting in the diagnosis of heart failure comprises:
(i) diagnosing or assisting in the diagnosis of whether the subject has heart failure; or
(ii) diagnosing or assisting in the diagnosis of a severity of heart failure in the subject; preferably, the severity refers to left ventricular ejection fraction or NYHA cardiac function classification.

3. The method of claim 2, wherein diagnosing or assisting in the diagnosis of whether a subject has heart failure comprises:
comparing the level of the N-terminal fragment of insulin-like growth factor binding protein-4 with a reference value, wherein
the subject has heart failure if the level of the N-terminal fragment of insulin-like growth factor binding protein-4 higher than the reference value;
the subject does not have heart failure if the level of the N-terminal fragment of insulin-like growth factor binding protein-4 lower than the reference value;
preferably, the reference value is about 148.5 ng/mL.

4. The method of any one of claims 1-3, diagnosing or assisting in the diagnosis of heart failure further comprises:
detecting a level of N-terminal pro-brain natriuretic peptide (NT-proBNP) in a sample from the subject;
diagnosing or assisting in the diagnosis of heart failure based on the level of the N-terminal fragment of insulin-like growth factor binding protein-4 and the level of the N-terminal pro-brain natriuretic peptide.

5. The method of claim 4, wherein diagnosing or assisting in the diagnosis of whether a subject has heart failure comprises:
comparing the level of the N-terminal fragment of IGFBP-4 and the level of the NT-proBNP with their respective reference values, and wherein:
the subject does not have heart failure if the level of the NT-proBNP is lower than its reference value;
the subject is at risk of heart failure and requires further determination if the level of the NT-proBNP is higher than its reference value, and the level of the N-terminal fragment of IGFBP-4 is lower than its reference value;
the subject has heart failure if the level of the N-terminal fragment of IGFBP-4 and the level of the NT-proBNP are both higher than their respective reference values;
preferably, the reference value of NT-proBNP is about 125 ng/L;
preferably, the reference value of NT-IGFBP-4 is about 220 ng/mL.

6. The method of claim 1, wherein assessing or assisting in the assessment of prognostic risk of heart failure comprises:
(i) assessing or assisting in the assessment of short-term prognostic risk in a patient with heart failure, the short-term referring to a period less than 30 days; or
(ii) assessing or assisting in the assessment of long-term prognostic risk in a patient with heart failure, the long-term referring to a period more than 30 days and less than 240 days;

7. The method of claim 6, wherein the prognostic risk refers to a risk of cardiac-related rehospitalization and/or death.

8. The method of any one of claims 1, 6 and 7, assessing or assisting in the assessment of prognostic risk of heart failure comprises comparing the level of the N-terminal fragment of insulin-like growth factor binding protein-4 with a reference value, wherein
the subject has a high risk of poor prognosis for heart failure if the level of the N-terminal fragment of insulin-like growth factor binding protein-4 higher than the reference value;
the subject has a low risk of poor prognosis for heart failure if the level of the N-terminal fragment of insulin-like growth factor binding protein-4 lower than the reference value;
preferably, the reference value for short-term prognostic risk is about 314 ng/mL;
preferably, the reference value for long-term prognostic risk is about 283 ng/mL.

9. The method of any one of claims 1 and 6-8, wherein assessing or assisting in the assessment of prognostic risk of heart failure further comprises:
detecting a level of N-terminal pro-brain natriuretic peptide (NT-proBNP) in a sample from the subject;
assessing or assisting in the assessment of prognostic risk of heart failure based on the level of the N-terminal fragment of insulin-like growth factor binding protein-4 and the level of the N-terminal pro-brain natriuretic peptide.

10. The method of claim 9, comprising:
comparing the level of the N-terminal fragment of IGFBP-4 and the level of the NT-proBNP with their respective reference values, wherein:
the subject has a high risk of poor prognosis for heart failure if the level of the N-terminal fragment of IGFBP-4 and the level of the NT-proBNP are both higher than their respective reference values;
the subject has a moderate risk of poor prognosis for heart failure if one of the level of the N-terminal fragment of IGFBP-4 and the level of the NT-proBNP is higher than its respective reference value;
the subject has a low risk of poor prognosis for heart failure if the level of the N-terminal fragment of IGFBP-4 and the level of the NT-proBNP are both lower than their respective reference values;
preferably, the reference value of NT-proBNP for short-term prognostic risk is about 3110 ng/L; preferably, the reference value of NT-IGFBP-4 for short-term prognostic risk is about 314 ng/mL;
preferably, the reference value of NT-proBNP for long-term prognostic risk is about 3110 ng/L; preferably, the reference value of NT-IGFBP-4 for long-term prognostic risk is about 283 ng/mL.

11. A method for assessing or assisting in the assessment of prognostic risk of coronary heart disease, wherein the method comprises:
detecting a level of N-terminal fragment of insulin-like growth factor binding protein-4 in a sample from a coronary heart disease patient, wherein the N-terminal fragment of insulin-like growth factor binding protein-4 is the N-terminal fragment obtained by catalytic hydrolysis of insulin-like growth factor binding protein-4 by pregnancy-associated plasma protein A;
assessing or assisting in the assessment of prognostic risk of coronary heart disease based on the level of N-terminal fragment of insulin-like growth factor binding protein-4.

12. The method of claim 11, comprising comparing the level of the N-terminal fragment of insulin-like growth factor binding protein-4 with a reference value, wherein
the subject has a high risk of poor prognosis for coronary heart disease if the level of the N-terminal fragment of insulin-like growth factor binding protein-4 higher than the reference value;
the subject has a low risk of poor prognosis for coronary heart disease if the level of the N-terminal fragment of insulin-like growth factor binding protein-4 lower than the reference value;
preferably, the reference value is about 174 ng/mL.

13. The method of any one of claims 1-12, wherein the level of N-terminal fragment of insulin-like growth factor binding protein-4 is detected by double-antibody sandwich immunoassay (e.g., double-antibody sandwich chemiluminescence immunoassay) comprising a capture antibody and a detection antibody, and wherein one of the capture antibody and the detection antibody is an antibody that specifically binds to NT-IGFBP-4, and the other is capable of recognizing and binding to NT-IGFBP-4 or full-length IGFBP-4;
preferably, the immunoassay is a double-antibody sandwich chemiluminescence immunoassay, and wherein the detection antibody is labelled with alkaline phosphatase, and the capture antibody is coated on the surface of a solid carrier, e.g., magnetic bead or microtiter plate.

14. A kit, comprising a first reagent for detecting N-terminal fragment of insulin-like growth factor binding protein-4 and a second reagent for detecting N-terminal pro-brain natriuretic peptide.

15. The kit of claim 14, wherein the first reagent is for double-antibody sandwich immunoassay (e.g., double-antibody sandwich chemiluminescence immunoassay) and comprises a capture antibody and a detection antibody, and wherein one of the capture antibody and the detection antibody is an antibody that specifically binds to NT-IGFBP-4, and the other is capable of recognizing and binding to NT-IGFBP-4 or full-length IGFBP-4; and/or, the second reagent is for immunoassay (e.g., enzyme-linked immunoassay, radioimmunoassay, fluorescent immunoassay, chemiluminescence immunoassay), preferably double-antibody sandwich immunoassay (e.g., double-antibody sandwich ELISA or CLIA);
preferably, the first reagent or the second reagent each independently comprises a capture antibody and a detection antibody, and wherein the detection antibody bears a detection label, e.g., radioactive label, fluorescent label, chemiluminescent label, biotin, colloidal gold, electrochemiluminescent label, quantum dot or enzyme (e.g., alkaline phosphatase); and/or, the capture antibody is coated on the surface of a solid carrier, e.g., magnetic bead or microtiter plate.
